# EUROPÄISCHE PATENTSCHRIFT

(11) **EP 1 163 323 B1**
(45) Veröffentlichungstag und Bekanntmachung des Hinweises auf die Patenterteilung: **20.07.2005**
(21) Anmeldenummer: 00912546.9
(22) Anmeldetag: 03.03.2000
(51) Int. Cl.: C12N 1/00, C12N 1/20, C12P 19/26

(54) **BIOTECHNOLOGISCHES VERFAHREN ZUR HERSTELLUNG VON N-ACETYLMANNOSAMIN**
BIOTECHNOLOGICAL METHOD FOR PRODUCING N-ACETYL MANNOSAMINE
PROCEDE BIOTECHNOLOGIQUE POUR LA PRODUCTION DE N-ACETYLMANNOSAMINE

(30) Priorität: 03.03.1999 EP 99104272; 20.07.1999 EP 99114065
(43) Veröffentlichungstag der Anmeldung: 19.12.2001
(73) Patentinhaber: Forschungszentrum Jülich GmbH, 52425 Jülich (DE)
(72) Erfinder: PETERSEN, Michael, CH-3930 Visp (CH); HEINZMANN, Klaus, CH-3932 Visperterminen (CH)
(74) Vertreter: Ritthaler, Wolfgang, Dr.rer.nat.Dipl.-Chem.
(86) Internationale Anmeldenummer: PCT/EP2000/001893
(87) Internationale Veröffentlichungsnummer: WO 2000/052138

(56) Entgegenhaltungen:
- EP-A- 0 428 947
- KUBOKI ET AL.: "An Expeditious Route to N-Glycolylneuraminic Acid Based on Enzyme-catalyzed Reaction." TETRAHEDRON, Bd. 53, Nr. 7, 1997, Seiten 2387-2400, XP004105332 in der Anmeldung erwähnt
- CHEMICAL ABSTRACTS, vol. 130, no. 14, 5. April 1999 (1999-04-05) Columbus, Ohio, US; abstract no. 179758, PLUMBRIDGE, JACQUELINE ET AL: "Convergent pathways for utilization of the amino sugars N-acetylglucosamine, N-acetylmannosamine, and N-acetylneuraminic acid by Escherichia coli" XP002143009 & J. BACTERIOL. (1999), 181(1), 47-54 ,

## Beschreibung

Die vorliegende Erfindung betrifft Mikroorganismen der Gattung Klebsiella, die befähigt sind, mit N-Acetylglucosamin als einziger C-Quelle zu wachsen, sowie ein biotechnologisches Verfahren zur Herstellung bzw. Anreicherung von N-Acetylmannosamin unter Verwendung dieser Mikroorganismen.

N-Acetylmannosamin (NAM) ist ein wichtiges Zwischenprodukt zur Herstellung von N-Acetylneuraminsäure (NANA), welche wiederum ein wichtiges Ausgangsmaterial zur Herstellung von therapeutischen Agentien ist (EP-A-0 701 623).

Zur Herstellung bzw. Anreicherung von NAM sind inzwischen mehrere biotechnologische Verfahren bekannt. Spivak C. T. & Roseman S. (JACS, Band 81 , 1959, S. 2403 - 2404) beschreiben die Anreicherung von NAM mittels gewaschenen Mikroorganismen der Spezies E. coli, die derart adaptiert wurden, dass sie mit N-Acetylglucosamin (NAG) als einziger C-Quelle wachsen können: Nachteile dieses Verfahrens sind, dass es grosstechnisch nicht gangbar ist und NAM nur in mässiger Ausbeute erhalten wird.

Sugai et al., (Tetrahedron, Vol. 53, 1997, S. 2387 - 2400) beschreiben ein Verfahren zur Anreicherung von NAM mittels gewaschenen Mikroorganismen der Spezies Rhodococcus rhodochrous, bei dem ausgehend von einer Mischung bestehend aus NAM und NAG in einem Verhältnis von 4,5 : 1 (82 : 18) NAM angereichert wird. Auch bei diesem Verfahren wird NAM nicht in wirtschaftlicher, industriell praktikabler Weise angereichert.

Aufgabe der vorliegenden Erfindung war es daher, Mikroorganismen bereit zu stellen, die für ein biotechnologisches Verfahren zur Herstellung bzw. Anreicherung von NAM eingesetzt werden können, bei dem das gewünschte Produkt schnell und auf einfache Weise angereichert wird.

Diese Aufgabe wird mit den erfindungsgemässen Mikroorganismen nach Anspruch 1 und mit dem Verfahren nach Anspruch 5 gelöst.

Die Erfindung betrifft somit Mikroorganismen der Gattung Klebsiella, welche befähigt sind, mit N-Acetyl-lucosamin als einziger C-Quelle zu wachsen und erhältlich sind durch:
(a) Selektion von Mikroorganismen, die mit N-Acetylglucosamin als einziger C-Quelle wachsen; und
(b) Selektion der in Stufe (a) erhaltenen Mikroorganismen auf Mikroorganismen, die befähigt sind, N-Acetylglucosamin in Gegenwart von N-Acetylmannosamin schneller als N-Acetylmannosamin zu metabolisieren

Die erfindungsgemässen Mikroorganismen können aus Bodenproben, Schlamm oder Abwasser unter Zuhilfenahme üblicher mikrobiologischer Techniken isoliert werden. Erfindungsgemäss erfolgt die Isolation dieser Mikroorganismen derart, dass man diese in einem Medium enthaltend NAG als einzige C-Quelle und mit einer geeigneten N-Quelle auf übliche Weise züchtet. Aus der durch Züchtung erhaltenen Kultur werden dann diejenigen Mikroorganismen selektioniert, die stabil sind und die Eigenschaft besitzen, NAG in Gegenwart von. NAM, beispielsweise äquimolarer Mengen NAM, schnell zu metabolisieren. Zweckmäßig werden unter den Mikroorganismen, die mit NAG als einziger C-Quelle wachsen können, diejenigen Mikroorganismen selektioniert, die nur sehr langsam oder gar nicht mit NAM wachsen können.

Zweckmäßig haben die in Stufe (b) selektionierten Mikroorganismen in einem Medium mit einer konstanten Konzentration von 10g/l NAG als einziger C- und Energiequelle bei 30°C eine Verdopplungszeit von < 10h, insbesondere von < 7,5h, bevorzugt von <5h und besonders bevorzugt von < 3h, und mit einer konstanten Konzentration von 10g/l NAM als einziger C- und Energiequelle bei 30°C eine Verdopplungszeit von > 24h.

Bevorzugt zeigen die selektionierten Mikroorganismen auch in Gegenwart hoher konstanter NAG-Konzentrationen, beispielsweise von 40-60 g/l, und in Gegenwart äquimolarer Mengen NAM die obigen Verdopplungszeiten von < 10h, insbesondere von < 7,5h, bevorzugt von <5h und besonders bevorzugt von < 3h.

Zweckmässig können die derart selektionierten Mikroorganismen NAG in einer Konzentration von 60 g/l in weniger als 30 h metabolisieren.

Als geeignete N-Quelle können die Mikroorganismen beispielsweise Ammonium verwenden.

Als Selektionsmedium können die in der Fachwelt üblichen Mineralsalzmedien verwendet werden, wie bspw. das in Tabelle 1 beschriebene Medium, das Mineralsalzmedium gemäss Kulla et al., Arch. Microbiol. (1983), 135, 1 - 7, oder niedermolare Puffer enthaltend Mineralsalze/Spurenelemente. Vorzugsweise wird das in Tabelle 1 beschriebene Medium verwendet. Als niedermolarer Puffer kann bspw. ein 10 - 100 mM Phosphatpuffer eingesetzt werden.

Üblicherweise erfolgt die Selektion bei einer Temperatur von 15 bis 60 °C, zweckmässig von 25 bis 45 °C, und bei einem pH-Wert zwischen pH 4 und pH 9, zweckmässig zwischen pH 6 und 8 .

Die erfindungsgemäßen Mikroorganismen sind Mikroorganismen der Gattung Klebsiella, zweckmässig der Spezies Klebsiella pneumoniae mit der Bezeichnung KHA (DSM 12702) und KHA1 (DSM 12703) bzw. deren "funktionell äquivalente Varianten". Die Mikroorganismen mit der Bezeichnung DSM 12702 und DSM 12703 wurden am 23.2.1999 bei der Deutschen Sammlung von Mikroorganismen und Zellkultur GmbH, Mascheroderweg 1b, D-38124 Braunschweig, gemäss Budapester Vertrag hinterlegt.

Unter "funktionell aequivalenten Varianten" werden Mikroorganismen verstanden, die im wesentlichen dieselben Eigenschaften und Funktionen wie die Ursprungsmikroorganismen besitzen. Derartige Varianten können zufällig, z. B. durch UV-Bestrahlung gebildet werden.

### Taxonomische Beschreibung von Klebsiella pneumoniae mit der Bezeichnung KHA (DSM 12702)

| | |
|---|---|
| Zellform | Stäbchen |
| Breite µm | 0,8-1,0 |
| Länge µm | 1,2-2,5 |
| Beweglichkeit | - |
| Gramm-Reaktion | - |
| Lyse durch 3% KOH | + |
| Aminopeptidase (Cerny) | + |
| Oxidase | - |
| Catalase | + |
| Säurebildung aus | |
| D-Glucose | + |
| D-Xylose | + |
| Erythrit | - |
| Adonit | - |
| D-Mannose | + |
| Rhamnose | + |
| Inosit | + |
| Saccharose | + |
| a-Methyl-D-glucosid | + |
| Inulin | - |
| Cellobiose | + |
| Maltose | + |
| Lactose | + |
| 5-Ketogluconat | + |
| L-Sorbose | + |
| Dulcit | + |
| β-Galactosidase | + |
| ADH (Alkoholdehydrogenase) | - |
| LDC (Lactatdecarboxylase) | - |
| ODC (Ornithindecarbxylase) | - |
| Voges Proskauer | + |
| Indol | - |
| Malonatverwertung | + |
| H₂S-Bildung | - |
| Citratverwertung (Simmons) | + |
| Phenylalanindesaminase | - |
| Urease (3 d) | + |
| Hydrolyse von | |
| Gelatine | - |
| DNA | - |
| Tween 80 | - |

### Taxonomische Beschreibung von Klebsiella pneumoniae mit der Bezeichnung KHA 1 (DSM 12703)

| | |
|---|---|
| Zellform | Stäbchen |
| Breite µm | 0,8-1,0 |
| Länge µm | 1,2-2,5 |
| Beweglichkeit | - |
| Gram-Reaktion | - |
| Lyse durch 3% KOH | + |
| Aminopeptidase (Cerny) | + |
| Oxidase | - |
| Catalase | + |
| Säurebildung aus | |
| D-Glucose | + |
| D-Xylose | + |
| Erythrit | - |
| Adonit | - |
| D-Mannose | + |
| Rhamnose | + |
| Inosit | + |
| Saccharose | + |
| a-Methyl-D-glucosid | + |
| Inulin | - |
| Cellobiose | + |
| Maltose | + |
| Lactose | + |
| 5-Ketogluconat | + |
| L-Sorbose | + |
| Dulcit | - |
| β-Galactosidase | + |
| ADH (Alkoholdehydroaenase) | - |
| LDC (Lactatdecarboxylase) | w |
| ODC (Ornithindecarboxylase) | - |
| Voges Proskauer | + |
| Indol | - |
| Malonatverwertung | + |
| H₂S-Bildung | - |
| Citratverwertung (Simmons) | + |
| Phenylalanindesaminase | - |
| Urease (3 d) | + |
| Hydrolose von | |
| Gelatine | - |
| DNA | - |
| Tween 80 | - |

Das erfindungsgemässe Verfahren zur Herstellung bzw. Anreicherung von NAM wird derart durchgeführt, dass man, ausgehend von einem NAG/NAM-Gemisch, NAG mittels den erfindungsgemässen Mikroorganismen fermentativ metabolisiert, wobei NAM angereichert wird.

Zweckmässig wird NAG als eine Mischung bestehend aus NAG und NAM in einem Gew.-Verhältnis von 1 : 1 eingesetzt. Dieses Mischungsverhältnis wird vorzugsweise gemäss R. Kuhn & G. Barschang (Liebigs Ann., 659, 1962, S. 156 - 163) derart erhalten, dass man das aus Chitin hydrolysierte NAG im basischen Milieu epimerisiert, wobei sich ein thermodynamisches Gleichgewicht von NAG zu NAM von 4 : 1 Gewichtsverhältnissen einstellt. Dann wird diese Mischung gemäss der oben beschriebenen Literaturstelle durch selektives Entfernen von NAG wie bspw. durch Kristallisation so angereichert, dass ein Mischungsverhältnis von NAM : NAG von 1 : 1 resultiert.

Prinzipiell ist die weitere erfindungsgemässe Anreicherung von NAM mit allen NAGmetabolisierenden Mikroorganismen möglich, die nach der bereits beschriebenen Selektionsmethode erhältlich sind. Insbesondere wird die Anreicherung mittels den Mikroorganismen Klebsiella pneumoniae mit der Bezeichnung KHA (DSM 12702) oder KHA1 (DSM 12703) oder mit deren "funktionell aequivalenten Varianten" durchgeführt.

Die Anreicherung von NAM erfolgt zweckmässig direkt durch Fermentation mit den selektionierten Mikroorganismen auf dem NAG/NAM-Gemisch (d. h. mit wachsenden Zellen unter aeroben Bedingungen), ohne vorheriges Abtrennen und Waschen der Mikroorganismen, z. B. durch Zentrifugation.

Als Fermentationsmedien können die gleichen wie die zuvor beschriebenen Medien, die zur Selektion eingesetzt werden, verwendet werden, indem als C-Quelle das NAG/NAM-Gemisch anstelle NAG verwendet wird.

Zweckmässig wird die Metabolisierung von NAG so durchgeführt, dass die Konzentration unter 20 Gew.%, vorzugsweise unter 10 Gew.%, liegt. Insbesondere erfolgt die Zugabe der entsprechenden NAG-NAM-Mischung einmalig, diskontinuierlich (in mehreren Portionen) oder kontinuierlich.

Der pH-Wert des Mediums kann in einem Bereich von 5 bis 9, vorzugsweise von 6 bis 8, liegen. Zweckmässig wird die Metabolisierung bei einer Temperatur von 20 bis 50 °C, vorzugsweise von 25 bis 40 °C, durchgeführt.

Nach einer üblichen Metabolisierung von unter 30 h kann das auf diese Weise angereicherte NAM durch übliche Aufarbeitungsmethoden wie z. B. durch Elektrodialyse, Filtrationstechniken und Kristallisation isoliert werden.

### Beispiel 1

### Anreicherung von Mikroorganismen, die mit NAG als einziger C-Quelle wachsen

100 ml Mineralsalzmedium (vgl. Tabelle 1) mit 2 g/l NAG wurden mit 2 g feuchtem Klärschlamm inokuliert und in einem 500-ml-Erlenmeyerkolben mit Schikanen bei 30 °C auf einem Schütteltisch inkubiert. Mit 5 ml dieser Suspension wurde ein weiterer Kolben mit 100 ml Mineralsalzmedium und 2 g/l NAG inokuliert und bei 30 °C auf einem Schütteltisch bebrütet. Nach 3 Tagen wurde eine weitere Passage durchgeführt.
Durch Ausplattieren einer Verdünnungsreihe und Reinigungsausstriche auf Agar-Platten des beschriebenen Anreicherungsmediums (bebrütet bei 30 °C) wurden diejenigen Mikroorganismen als Einzelkolonien erhalten, die auf NAG als einziger C-Quelle wuchsen.

### Beispiel 2

### Selektion von Mikroorganismen, die mit NAG als einziger C-Quelle wachsen, nicht jedoch mit NAM

Mikroorganismen, die mit NAG als einziger C-Quelle wachsen (z.B. aus der Anreicherung gemäss Beispiel 1), wurden auf Agar-Platten mit Mineralsalzmedium (vgl. Tabelle 1) und 5 g/l NAG bzw.
Mineralsalzmedium (vgl. Tabelle 1) und 5 g/l NAM ausgestrichen und bei 30 °C bebrütet. Die Mikroorganismen, die sehr schnell auf NAG-Platten wuchsen, jedoch nicht oder nur sehr langsam auf NAM-Platten, wurden selektiert.

### Beispiel 3

### Selektion auf schnelles Wachstum in Gegenwart höherer NAG-Konzentrationen

100 ml Mineralsalzmedium (vgl. Tabelle 1) mit 5 g/l, 10 g/l, 20 g/l und 40 g/l NAG wurden mit den gemäß Beispiel 2 selektionierten Mikroorganismen von einer Agar-Platte (mit NAG gemäss Bsp. 2) inokuliert und in einem 500-ml-Erlenmeyerkolben mit Schikanen bei 30 °C auf einem Schütteltisch bebrütet. Durch Kontrolle der optischen Dichte konnte die Wachstumsgeschwindigkeit unter den verschiedenen Bedingungen untersucht werden. Stämme, die sehr schnell und in Gegenwart höherer NAG-Konzentrationen wuchsen, wurden selektioniert. Die Stämme wurden wurden ferner auf ihr Wachstum in Gegenwart höherer und äquimolarer NAM-Konzentrationen getestet. Auf diese Weise wurden u.a. die beiden Stämme KHA (DSM 12702) und KHA1 (DSM 12703) selektioniert.

| Stamm KHA | OD(650) nach 18h | OD(650) nach 42h |
|---|---|---|
| 5 g/l NAG | 4,2 | 4,5 |
| 10 g/l NAG | 6,5 | 8,5 |
| 20 g/l NAG | 8,2 | 13,8 |
| 40 g/l NAG | 9,1 | 14,7 |

### Beispiel 4

### Herstellung eines NAG/NAM-Gemisches im Verhältnis 1:1

1 kg NAG wurde in 3 l Wasser gelöst, der pH > 11 mittels 30 %iger Natronlauge eingestellt, und 1-2 Tage bei 20-40 °C stehen gelassen, bis ein Verhältnis von NAG / NAM von ca. 4:1 erreicht war. Die Lösung wurde mit Schwefelsäure neutralisiert und im Vakuum auf ca. 30 % konzentriert. Dabei kristallisierten 0,6 kg NAG, die abfiltriert wurden und erneut für die gleiche Reaktion eingesetzt werden konnten. Das Filtrat (0,8 - 1 l) enthielt 0,4 kg NAG / NAM im Verhältnis 1:1 (laut GC-Analyse).

### Beispiel 5

### Einfaches Batch-Verfahren

2 l Mineralsalzmedium (vgl. Tabelle 1) mit 40 g/l NAG/NAM-(1:1)-Gemisch wurden mit 80 ml einer Vorkultur des Stammes KHA bzw. KHA1 (über Nacht gewachsen auf Mineralmedium mit 5 g/l NAG; OD > 4) inokuliert und bei pH 7, 30 °C, unter Belüftung und Rühren fermentiert. Nach 16 h betrug die OD(650 nm) 13,7 (Stamm KHA) bzw. 15,5 (Stamm KHA1), und es konnte im Medium praktisch kein oder nur noch Spuren NAG nachgewiesen werden, wohl aber NAM (GC-Analytik; NAG : NAM < 5 : 95).

### Beispiel 6:

### Fed-Batch-Verfahren

1,5 l Mineralsalzmedium (vgl. Tabelle 1) mit 40 g/l NAG/NAM-(1:1)-Gemisch wurden mit 100 ml einer Vorkultur des Stammes KHA (über Nacht gewachsen auf Mineralmedium mit 10 g/l NAG; OD > 4) inokuliert und bei pH 7, 30°C, unter Belüftung (1,5 l/min Luft) und Rühren (500 rpm) fermentiert. Nach 14 h wurden 360 ml einer 46 %-igen Lösung NAG/NAM-(1:1)-Gemisch zugegeben (total: 113 g NAG, 113 g NAM; entsprach je 57g/l). Nach 29,5 h betrug die OD(650nm) 38,5, und es konnte im Medium praktisch kein oder nur noch Spuren NAG nachgewiesen werden, wohl aber NAM (GC-Analytik; NAG : NAM < 5 : 95).

### Beispiel 7

### Fed-Batch-Verfahren, NAG/NAM-(2:1)-Gemisch

1,5 l Mineralsalzmedium (vgl. Tabelle 1) mit 40 g/l NAG/NAM-(2:1)-Gemisch wurden mit 100 ml einer Vorkultur des Stammes KHA1 (über Nacht gewachsen auf Mineralmedium mit 10 g/l NAG; OD > 4) inokuliert und bei pH 7, 30 °C, unter Belüftung (1,5L/min Luft) und Rühren (500 rpm) fermentiert. Nach 16 h wurden 240 ml einer 46 %-igen Lösung NAG/NAM-(2:1)-Gemisch zugegeben (total: 114 g NAG = 60 g/l, 57 g NAM = 30 g/l). Nach 22,5 h betrug die OD(650nm) 43,4, und es konnten im Medium praktisch kein oder nur noch Spuren NAG nachgewiesen werden, wohl aber NAM (GC-Analytik; NAG : NAM < 5 : 95).

**Tabelle 1**

| **Mineralsalzmedium (pH 7,0)** |
|---|
| 2,0 g/l (NH₄)₂SO₄, |
| 2,0 g/l Na₂HPO₄, |
| 1,0 g/l KH₂PO₄, |
| 2,0 g/l NaCl, |
| 0,4 g/l MgCl₂ x 6H₂O, |
| 14,5 mg/l CaCl₂ x 2H₂O, |
| 0,8 mg/l FeCl₃ x 6H₂O, |
| 0,1 mg/l ZnSO₄ x 7H₂O, |
| 0,09 mg/l MnCl₂ x 4H₂O, |
| 0,3 mg/l H₃BO₄, |
| 0,2 mg/l CoCl₂ x 6H₂O, |
| 0,01 mg/l CuCl₂ x 2H₂O, |
| 0,02 mg/l NiCl₂ x 6H₂O, |
| 0,03 mg/l NaMoO₄ x 2H₂O, |
| 5,0 mg/l EDTA x 2Na x 2H₂O, |
| 2,0 mg/l FeSO₄ x 7H₂O |

## Patentansprüche

1. Mikroorganismen der Gattung Klebsiella, welche befähigt sind, mit N-Acetylglucosamin als einziger C-Quelle zu wachsen, erhältlich durch:
(a) Selektion von Mikroorganismen, die mit N-Acetylglucosamin als einziger C-Quelle wachsen; und
(b) Selektion der in Stufe (a) erhaltenen Mikroorganismen auf Mikroorganismen, die befähigt sind, N-Acetylglucosamin in Gegenwart von N-Acetylmannosamin schneller als N-Acetylmannosamin zu metabolisieren.

2. Mikroorganismen nach Anspruch 1, **dadurch** erhältlich, daß die Selektion der in Stufe (a) erhaltenen Mikroorganismen in Stufe (b) auf Mikroorganismen erfolgt, die nicht mit N-Acetylmannosamin wachsen können.

3. Mikroorganismen nach einem der Ansprüche 1 oder 2, **dadurch gekennzeichnet, dass** sie N-Acetylglucosamin in einer Konzentration von 60 g/l in weniger als 30 h metabolisieren.

4. Mikroorganismen nach einem der Ansprüche 1 bis 3 der Spezies Klebsiella pneunomiae, insbesondere Mikroorganismen mit der Bezeichnung KHA (DSM 12702) und KHA1 (DSM 12703).

5. Biotechnologisches Verfahren zur Herstellung von N-Acetylmannosamin umfassend die fermentative Metabolisierung von N-Acetylglucosamin aus einem Gemisch mit N-Acetylmannosamin mittels den Mikroorganismen nach einem der Ansprüche 1 bis 4, wobei N-Acetylmannosamin angereichert wird.

6. Verfahren nach Anspruch 5, **dadurch gekennzeichnet, dass** die Mikroorganismen nach der Selektion und Anzucht direkt für die fermentative Metabolisierung eingesetzt werden.

7. Verfahren nach einem der Ansprüche 5 oder 6, **dadurch gekennzeichnet, dass** die fermentative Metabolisierung mittels Mikroorganismen der Spezies Klebsiella pneunomiae, insbesondere den Mikroorganismen mit der Bezeichnung KHA (DSM 12702) oder KHA1 (DSM 12703) oder mit deren funktionell äquivalenten Varianten durchgeführt wird.

8. Verfahren nach einem der Ansprüche 5 bis 7, **dadurch gekennzeichnet, dass** die fermentative Metabolisierung bei einer Temperatur von 20 bis 50 °C und bevorzugt bei einem pH-Wert von 5 bis 9 durchgeführt wird.

## Claims

1. Microorganisms of the genus Klebsiella that are capable of growing with N-acetylglucosamine as the only carbon source, obtainable by:
(a) selecting microorganisms that grow with N-acetylglucosamine as the only carbon source; and
(b) selecting the microorganisms obtained in step (a) for microorganisms that are capable of metabolising N-acetylglucosamine in the presence of N-acetylmannosamine more rapidly than N-acetylmannosamine.

2. Microorganisms according to claim 1, obtainable in that the selection of the microorganisms obtained in step (a) is carried out in step (b) for microorganisms that are not able to grow with N-acetylmannosamine.

3. Microorganisms according to either claim 1 or claim 2, **characterised in that** they metabolise N-acetylglucosamine in a concentration of 60 g/l in less than 30 hours.

4. Microorganisms according to any one of claims 1 to 3 of the species Klebsiella pneunomiae, especially microorganisms having the designation KHA (DSM 12702) and KHA1 (DSM 12703).

5. Biotechnological process for the preparation of N-acetylmannosamine, which comprises metabolising N-acetylglucosamine by fermentation from a mixture comprising N-acetylmannosamine by means of the microorganisms according to any one of claims 1 to 4, wherein N-acetylmannosamine is enriched.

6. Process according to claim 5, **characterised in that** the microorganisms are used directly for the metabolisation by fermentation after selection and culturing.

7. Process according to either claim 5 or claim 6, **characterised in that** the metabolisation by fermentation is carried out by means of microorganisms of the species Klebsiella pneunomiae, especially the microorganisms having the designation KHA (DSM 12702) or KHA1 (DSM 12703) or with functionally equivalent variants thereof.

8. Process according to any one of claims 5 to 7, **characterised in that** the metabolisation by fermentation is carried out at a temperature of from 20 to 50°C and preferably at a pH value of from 5 to 9.

## Revendications

1. Micro-organismes de l'espèce Klebsiella, qui sont capables de croître à l'aide de N-acétylglucosamine en tant que source unique de C, que l'on peut obtenir:
a) par sélection de micro-organismes, qui croissent avec la N-acétylglucosamine en tant que source unique de C; et
b) par sélection des micro-organismes obtenus dans l'étape (a) en vue d'obtenir des micro-organismes qui sont capables de métaboliser la N-acétylglucosamine en présence de N-acétylmannosamine plus rapidement que la N-acétylmannosamine.

2. Micro-organismes selon la revendication 1, que l'on peut obtenir en ce que la sélection des micro-organismes obtenus dans l'étape (a) se fait dans l'étape (b) en vue d'obtenir des micro-organismes qui ne croissent pas avec la N-acétylmannosamine.

3. Micro-organismes selon l'une quelconque des revendications 1 ou 2, **caractérisés en ce qu'**ils métabolisent la N-acétylglucosamine dans une concentration de 60 g/l en moins de 30 heures.

4. Micro-organismes selon l'une quelconque des revendications 1 à 3 de l'espèce Klebsiella pneunomiae, en particulier micro-organismes ayant la désignation KHA (DSM 12702) et KHAI (DSM 12703).

5. Procédé biotechnologique en vue de la fabrication de N-acétylmannosamine, comprenant la métabolisation par fermentation de la N-acétylglucosamine à partir d'un mélange avec la N-acétylmannosamine, à l'aide de micro-organismes selon l'une quelconque des revendications 1 à 4, dans lequel la N-acétylmannosamine est enrichie.

6. Procédé selon la revendication 5, **caractérisé en ce que** les micro-organismes après la sélection et la culture sont directement utilisés pour la métabolisation par fermentation.

7. Procédé selon l'une quelconque des revendications 5 ou 6, **caractérisé en ce que** la métabolisation par fermentation est effectuée à l'aide de micro-organismes de l'espèce Klebsiella pneunomiae, en particulier à l'aide de micro-organismes ayant la désignation KHA (DSM 12702) ou KHAI (DSM 12703) ou à l'aide de leurs variantes fonctionnellement équivalentes.

8. Procédé selon l'une quelconque des revendications 5 à 7, **caractérisé en ce que** la métabolisation par fermentation est effectuée à une température de 20 à 50 °C et, de préférence, à un pH de 5 à 9.
